Europäisches Patentamt

European Patent Office

Office européen des brevets

(19)

(11) Veröffentlichungsnummer : **0 076 506**
**B1**

(12) **EUROPÄISCHE PATENTSCHRIFT**

(45) Veröffentlichungstag der Patentschrift :
03.04.85

(21) Anmeldenummer : 82109156.8

(22) Anmeldetag : 04.10.82

(51) Int. Cl.⁴ : **C 12 Q   1/38, G 01 N  33/48**

(54) Verfahren zur Bestimmung der Aktivität von Chymotrypsin oder Trypsin im Stuhl.

(30) Priorität : 06.10.81 DE 3139719

(43) Veröffentlichungstag der Anmeldung :
13.04.83 Patentblatt 83/15

(45) Bekanntmachung des Hinweises auf die Patenterteilung : 03.04.85 Patentblatt 85/14

(84) Benannte Vertragsstaaten :
AT BE CH DE FR GB IT LI LU NL SE

(56) Entgegenhaltungen :
EP-A- 0 008 338
US-A- 3 886 136
ANALYTICAL BIOCHEMISTRY, Band 99, 1979 New
York E.G. DELMAR et al. "A sensitive new substrate
for chymotrypsin" Seiten 316 bis 320

(73) Patentinhaber : BOEHRINGER MANNHEIM GMBH
Patentabteilung, Abt. E Sandhofer Strasse 112-132
Postfach 31 01 20
D-6800 Mannheim 31 Waldhof (DE)

(72) Erfinder : Möller, Gerald, Dr.
Waxensteinstrasse 17
D-8132 Tutzing (DE)
Erfinder : Kaspar, Klaus Peter, Dr.
Gröberweg 1
D-8132 Tutzing (DE)

(74) Vertreter : Weickmann, Heinrich, Dipl.-Ing. et al
Patentanwälte Dipl.-Ing. H.Weickmann Dipl.-Phys.Dr.
K.Fincke Dipl.-Ing. F.A.Weickmann Dipl.-Chem. B.
Huber Dr.-Ing. H. Liska Dipl.-Phys.Dr. J. Prechtel
Postfach 860820
D-8000 München 86 (DE)

## Beschreibung

Bei Verdacht auf eine chronische Pankreatitis als auch auf Mukoviscidose bei Säuglingen ist die Diagnose einer Pankreasinsuffizienz ein wichtiger Parameter, dessen klinisch-chemischer Nachweis bis heute noch nicht in befriedigender Weise gelöst werden konnte. Als verläßlichste Methode hierfür wird allgemein der Pankreozym-Sekretin-Test angesehen. Hierbei wird nach Stimulation durch Pankreozymin und Sekretin mittels einer Sonde das Pankreassekret aufgefangen und anschließend aus folgende Parameter untersucht : Volumen, Bicarbonatkonzentration-Aktivitäten von Amylase, Lipase, Trypsin und Chymotrypsin. Der Hauptnachteil dieser Methode ist der hohe zeitliche und technische Aufwand sowie die große Belästigung für den Patienten.

Eine weitere Methode ist, sowohl als Suchtest als auch zur Verlaufskontrolle der chronischen Pankreatitis und der Mukoviscidose, die Bestimmung von Chymotrypsin oder Trypsin im Stuhl. Im fall einer chronischen Pankreatitis und bei Mukoviscidose sind die Aktivitätswerte von Trypsin und Chymotrypsin erniedrigt, wobei sich die Chymotrypsinwerte als die besseren Parameter erwiesen haben. Zur Bestimmung von Chymotrypsin oder Trypsin im Stuhl wird eine Stuhlsuspension mit einer wäßrigmethanolischen Lösung eines spezifischen Substrats versetzt und die in der Zeiteinheit freigesetzte Aminosäure bestimmt. Die Bestimmung der freigesetzten Aminosäure kann z. B. durch Titration mittels Lauge, insbesondere Natronlauge, erfolgen (pH-stat-Verfahren, Haverback et al., Gastroenterology *44* (1963), 588-597 ; Ammann, Fortschritte in der Pankreasfunktionsdiagnostik, Springer Verlag, Berlin, Heidelberg, New York (1967)), oder es wird die Zeit gemessen, die zur Erniedrigung des pH-Werts der Lösung um 0,1 Einheiten führt (pH-drop-Verfahren, vgl. Robinson, Smith, Elliott, Clin. Chim. Acta *62* (1975), 225-229).

Alle bekannten Methoden zur Bestimmung der Aktivität von Chymotrypsin oder Trypsin im Stuhl besitzen jedoch den Nachteil eines hohen apparativen und zeitlichen Aufwands. Eine rasche und einfache Bestimmung der Aktivität auf photometrischem Wege war bisher nicht möglich : Für eine einigermaßen genaue Bestimmung sind bei sehr kleinem Probeneinsatz die zu bestimmenden Meßgrößen zu gering, bei höherem Probeneinsatz die Eigenfärbung und Trübung durch Suspensions-Partikel zu stark. Eine photometrische Bestimmung nach einer Zentrifugation der Stuhlsuspension ist deshalb nicht möglich, weil das Enzym relativ fest an die Stuhlpartikel gebunden ist und sich nach der Zentrifugation fast vollständig im Sediment befindet oder sich nur unvollständig und nicht reproduzierbar in Lösung begibt.

Aufgabe der Erfindung ist deshalb die Schaffung eines Verfahrens zur raschen, einfachen, genauen und gut reproduzierbaren Bestimmung der Aktivität von Chymotrypsin oder Trypsin im Stuhl. Diese Aufgabe wird mit dem erfindungsgemäßen Verfahren zur Aktivitätsbestimmung gelöst.

Gegenstand der Erfindung ist ein Verfahren zur Bestimmung der Aktivität von Chymotrypsin oder Trypsin im Stuhl durch Messung der Geschwindigkeit des Spaltung eines geeigneten Substrats durch eine Stuhlsuspension in wäßrigem oder wäßrig-organischem Medium, dadurch gekennzeichnet, daß man den Stuhl in Gegenwart eines oberflächenaktiven Mittels suspendiert.

Die Messung der Geschwindigkeit der Spaltung des Substrats kann nach einer der aus dem Stand der Technik dafür bekannten Methoden erfolgen, wie z. B. durch Titration der freigesetzten Aminosäure mittels Lauge (pH-stat-Verfahren). Dabei hat es sich gezeigt, daß bei der erfindungsgemäßen Durchführung des Verfahrens in Gegenwart eines oberflächenaktiven Mittels im allgemeinen mehr als 90 % der Enzymaktivität solubilisiert sind, die Reaktionsgeschwindigkeit der Substratspaltung beträchtlich erhöht und der scheinbare Km-Wert des Substrats herabgesetzt ist (Aktivierungsfaktor ca. 2 bis 10), wodurch die bisher auftretenden Probleme (insbesondere Bindung des Enzyms an die Stuhlpartikel und überraschend große Km-Werte für unbehandelte Stuhlproben im Vergleich zu kristallinem Enzym bei bestimmten Substraten) überwunden werden können. Insbesondere ist es dadurch möglich, die Geschwindigkeit der Spaltung des Substrats auch auf photometrischem Wege rasch, einfach, genau und mit geringem Substrateinsatz zu messen. Die mit dem erfindungsgemäßen Verfahren in Gegenwart eines oberflächenaktiven Mittels erhaltenen Ergebnisse sind insbesondere auch deshalb überraschend, weil bei reinem α-Chymotrypsin aus Rinderpankreas praktisch kein Unterschied bei der Durchführung der Bestimmungsmethode in oder ohne Gegenwart eines oberflächenaktiven Mittels festgestellt wurde.

Als oberflächenaktives Mittel kann im Prinzip jedes geeignete Tensid eingesetzt werden, wie anionische oder ampholytische Tenside, und vorzugsweise nichtionogene Tenside und insbesondere kationische Tenside.

Anionische Tenside sind zum Beispiel Alkansulfonate, Olefinsulfonate, zum Beispiel Cumensulfonat, Estersulfonate, Alkylarylsulfonate insbesondere Alkylbenzolsulfonate vom Typ des Dodecylbenzolsulfonats und Alkylnaphthalinsulfonate, Alkylsulfate, wie zum Beispiel Natrium-laurylsulfat, Äthersulfate oder Fettalkoholsulfate, Salze von Fettsäuren und Cholsäuren ; ampholitische Tenside sind solche mit anionenaktiven und kationenaktiven hydrophilen Gruppen, wie zum Beispiel Glycerinderivate mit Betain-Struktur, Sulfobetaine und Lecithine ; nichtionogene Tenside sind zum Beispiel Polyäther, insbesondere Alkylphenolpolyglykoläther und andere Produkte der Äthoxylierung von Fettsäuren, Fettsäureamiden, Fettaminen und Fettalkoholen, zum Beispiel äthoxylierter Laurylalkohol, Polymere aus Propylen und Äthylenoxid, Polyoxyäthylen-alkyläther oder -nonylphenyläther, Polyoxyäthylen-sorbitan-mono-oleat oder Laureat, Additionsprodukte aus Propylenoxid/Äthylendiamin/Äthylenoxid, Aminoxide und Fettsäureester von Polyalkoholen, Talg-Alkohol-Polyglykoläther ; kationische Tenside sind zum Beispiel geradkettige und cyclische Ammoniumverbindungen, z. B. N-Cetyl-N-äthyl-morpholinmetosulfat, Benzalkoniumchloride und andere quartäre Ammoniumsalze, Aminsalze, Pyridiniumsalze, Pyridiniumsalze oder quarternäre Fettamin-polyglykoläther.

Die Wahl des am besten geeigneten Tensids ist auch von den übrigen Reaktionsbedingungen abhängig, insbesondere von der Art des Enzyms und Substrats, von der Art und Konzentration der Salze, aber auch vom Ph-Wert des Mediums.

Von den für das erfindungsgemäße Verfahren in erster Linie geeigneten kationischen Detergentien zeigen den stärksten Aktivierungseffekt quartäre Ammoniumverbindungen, vorzugsweise der Formel $R_1R_2N^{\oplus}(CH_3)_2$, worin $R_1$ vorzugsweise einen Alkylrest mit 8 bis 14 Kohlenstoffatomen, und insbesondere Lauryl und Cetyl bedeutet, und $R_2$ vorzugsweise einen Niedralkylrest mit 1 bis 5 Kohlenstoffatomen oder einen Aralkylrest, oder auch einen Hydroxyalkylrest, und insbesondere Methyl oder Benzyl, und Alkylpyridiniumsalze mit vorzugsweise 12 bis 18 Kohlenstoffatomen im Alkylrest, wie z. B. Laurylpyridiniumchlorid, Laurylpyridiniumdisulfat, und insbesondere das Hexadecylpyridiniumchlorid. Ein für das erfindungsgemäße Verfahren besonders gut geeignetes Tensid ist das Lauryl-trimethyl-ammoniumchlorid.

Die Konzentration des oberflächenaktiven Mittels in der zur Suspension des Stuhls verwendeten Homogenisierlösung beträgt im allgemeinen ca. 0,02 bis ca. 10 Gew.-%, vorzugsweise 0,5 bis 5 Gew.-%. Die Konzentration des oberflächenaktiven Mittels in der Meßlösung (Stuhlsuspension und Substratlösung) sollte zweckmäßigerweise ca. 0,0005 bis 0,5, und vorzugsweise 0,01 bis 0,3 Gew.-% betragen. Die Konzentration der Stuhlprobe in der Suspension beträgt z. B. bei Verwendung des Substrats Succ-Ala-Ala-Pro-Phe-pNA 0,2 bis 2 %.

Vorzugsweise enthält die zur Suspension des Stuhls verwendete Homogenisierlösung neben dem oberflächenaktiven Mittel noch ein oder mehrere wasserlösliche Salze, wie z. B. Alkali- und/oder Erdalkalichloride oder Sulfat. Als besonders zweckmäßig hat sich ein Gehalt an NaCl von 100 bis 1 000 mMol/l oder ein Gehalt an $CaCl_2$ von 20 bis 500 mMol/l oder eine Kombination beider Salze in den genannten Konzentrationsbereichen erwiesen. Es eignen sich aber auch organische Salze, z. B. Acetate oder Citrate, sowie Salze anderer Kationen. Die Salze sollten zweckmäßig in einer Konzentration vorliegen, die einer Ionenstärke von 20 bis 1 000 mVal/l entspricht.

Dabei wurde überraschend festgestellt, daß durch eine ein oberflächenaktives Mittel und Salze (Ionenstärke) enthaltende Homogenisierlösung eine überadditive Steigerung der Enzimaktivität (Erhöhung der Reaktionsgeschwindigkeit) eintritt, d. h. die Steigerung ist größer als die Summe der in Gegenwart von oberflächenaktivem Mittel oder in Gegenwart von Salzen erhaltenen Werte. Des weiteren bringt erst die Kombination von Salz und Detergens — im Gegensatz zu den Einzelkomponenten — einen konstant hohen und repräsentativen Teil des partikelgebundenen Enzyms in Lösung.

Als Substrat kann im allgemeinen jedes aus dem Stand der Technik für die Bestimmung der Aktivität von Chymotrypsin oder Trypsin im Stuhl nach den bisherigen Methoden als geeignet bekanntes Substrat verwendet werden (vgl. z. B. Grossman, Proc. Soc. Biol. Med. *110* (1962), 41 ; Del Mar et al., Anal. Biochem. *99* (1979) 316 ; Nakajama et al., J. Biol. Chem. *254* (10), 1979, (4027-4032). Für die Chymotrypsin-Bestimmung als besonders geeignet, vor allem im Hinblick auf Wasserlöslichkeit, Stabilität, Km-Wert und Geschwindigkeitskonstante, erwiesen sich das Ala-Ala-Phe-pNA und insbesondere das Succ-Ala-Ala-Pro-Phe-pNA und MeO-Succ-Arg-Pro-Tyr-pNA ; sie eignen sich insbesondere sehr gut für eine photometrische Bestimmung. Ein für die Bestimmung der Trypsin-Aktivität gut geeignetes Substrat ist z. B. Chromozym TRY® (Carbobenzoxy-valyl-glycyl-arginin-p-nitroanilid-acetat).

Zur Herstellung der Reagenslösung wird das Substrat in Wasser oder einem Gemisch aus Wasser und einem organischen Lösungsmittel gelöst. Das organische Lösungsmittel dient dabei als Lösungsvermittler für das Substrat, und ist z. B. Acetonitril, Dimethylsulfoxid, Aceton oder Methanol. Die Reagenslösung enthält vorzugsweise auch noch die gleichen Salze wie sie für die Homogenisierlösung verwendet werden ; die Konzentration dieser Salze in der Reagenslösung ist im allgemeinen niedriger als die Konzentration in der Homogenisierlösung : die gesamte Salzkonzentration beträgt vorzugsweise ca. 10 bis 500 mMol/l, z. B. 250 mMol/l Natriumchlorid und 20 mMol/l Calciumchlorid.

Zur Durchführung der Messung wird eine bestimmte Menge einer Suspension der Stuhlprobe in der Homogenisierlösung oder des durch Zentrifugation bis zur Klärung erhaltenen Zentrifugation bis zur Klärung erhaltenen Zentrifugationsüberstands zu einer bestimmten Menge der Reagenslösung zugegeben (z. B. 100 µl Probensuspension zu 2 ml Reagenslösung) und die Geschwindigkeit der Spaltung z. B. titrimetrisch oder photometrisch bestimmt. Die Menge an Probensuspension (Probenverdünnung) richtet sich dabei zur Erzielung gut meßbarer Werte, z. B. gut meßbarer Extinktionsanstiege/Minute, vor allem nach der zu erwartenden Enzymaktivität. Aufgrund der Empfindlichkeitssteigerung durch Einsatz des Detergens kann die Probenmenge so klein gewählt werden, daß die Eigenabsorption der suspendierten Festkörper gering ist und somit eine photometrische Messung mit der Suspension erst ermöglicht wird. Der pH-Wert der Homogenisierlösung ist nicht besonders kritisch ; er liegt im allgemeinen zwischen 3 und 10, und insbesondere zwischen 6 und 8. Der pH-Wert der Mischung aus Probensuspension und Reagenslösung liegt im allgemeinen zwischen 8 und 10, und vorzugsweise bei pH = 9 ; diese Werte werden vorzugsweise über den pH-Wert der Reagenslösung eingestellt. Es kann auch vorteilhaft sein, den Lösungen zur Einstellung des pH-Werts ein geeignetes Puffergemisch zuzugeben, wie z. B. Tris-Puffer, Glycin-Puffer oder Glycylglycin-Puffer. Die Pufferkonzentration liegt im allgemeinen zwischen 10 und 1 000 mMol/l, insbesondere bei 50 bis 200 mMol/l.

Liegen sehr geringe Chymotrypsinmengen vor, wie sie beispielsweise auftreten, wenn Dosierungsvorrichtungen verwendet werden, welche nur die in einer Grenzfläche vorhandene Chymotrypsinmenge zu erfassen gestatten, so ist es zweckmäßig, eine besonders empfindliche Nachweisreaktion anzuwenden. Eine derartige empfindliche Reaktion ist in J. Biol. Chem. *128* (1939) 537, beschrieben und als Bratton/Marshall-Reaktion bekannt. Sie beruht auf dem Zusatz von Natriumnitrit und N-$\alpha$-Naphthyläthylendiamin und anschließenden Säurezusatz. Als Säure wird dabei vorzugsweise Trichloressigsäure oder eine Säure vergleichbarer Stärke eingesetzt.

Die Messung kann manuell oder mit einem automatischen Analysengerät durchgeführt werden. Für die photometrische Aktivitätsbestimmung, und insbesondere zur automatischen photometrischen Messung (Bestimmung der Extinktion) ist es zweckmäßig, die Probensuspension vor der Messung bis zur Klärung zu zentrifugieren.

Als besonders zweckmäßig für die Durchführung des erfindungsgamäßen Verfahrens hat sich die in der deutschen Patentanmeldung P 31 39 702.6 unter der Bezeichnung « Gefäß zur Handhabung von pastösem Probenmaterial » der Anmelderin beschriebene Vorrichtung erwiesen. Mit dem erfindungsgemäßen Verfahren ist eine einfache und ästhetische Probenahme, Dosierung, Aufarbeitung und Messung möglich, wodurch werder Patient noch Laborpersonal belastet werden.

Die nachfolgenden Beispiele erläutern die Erfindung näher, ohne sie darauf zu beschränken. Wenn nicht anders angegeben, beziehen sich Prozentangaben auf Gewichtsprozent.

## Beispiel 1

a) Herstellung der Homogenisierlösung :

| | |
|---|---|
| NaCl | : 2,9 g (= 500 mmol/l) |
| CaCl$_2$ | : 1,1 g (= 100 mmol/l) |
| 33 %ig. Lauryl-trimethyl-ammoniumchlorid | : 2,0 g (= 0,7 %) |
| H$_2$O dest. | : ad 100 ml |

b) Herstellung der Reagenzlösung :

| | |
|---|---|
| Succ-Ala-Ala-Pro-Phe-pNA | 29,5 mg (0,5 mmol/l) |
| NaCl | 1,46 g (250 mmol/l) |
| CaCl$_2$ | 222 mg (20 mmol/l) |
| Puffer Tris/HCl, pH 9,0, 60 mmol/l | ad 100 ml |

c) Homogenisierung der Probe :

Ca. 100 mg Stuhlprobe werden mit der 100-fachen Gewichtsmenge an Homogenisierlösung versetzt und in einem geeigneten Gerät zu einer feinen Suspension verarbeitet.

d) Durchführung der Messung :

In eine 1 cm Meßküvette werden 2 ml der Reagenzlösung pipettiert, auf 25 °C temperiert und mit 100 µl des Probenhomogenisats versetzt. Nach kurzer Durchmischung wird die Extinktionszunahme / min. bei 405 nm bestimmt. Zur Berechnung der Enzymaktivität / g Stuhl ist der Extinktionsanstieg / min. bei 405 nm mit dem Faktor 212 zu multiplizieren.

## Beispiel 2

Die Herstellung der Homogenisierlösung und der Reagenzlösung erfolgt gemäß Beispiel 1, ebenso die Homogenisierung der Probe. Im Anschluß an die Homogenisierung wird die Suspension bis zur Klärung der Lösung zentrifugiert. Vom Zentrifugationsüberstand wird ein Aliquot entnommen und entweder manuell wie in Beispiel 1 oder unter Einsatz eines automatischen Analysengeräts die Enzymaktivität bestimmt.

## Beispiel 3

a) Herstellung der Homogenisierlösung : Es wird eine wäßrige Lösung mit folgenden Bestandteilen hergestellt :

| | | |
|---|---|---|
| Tris/HCl-Puffer, pH 9,0 | 60 | mmol/l |
| NaCl | 250 | mmol/l |
| CaCl$_2$ | 20 | mmol/l |
| Lauryl-trimethyl-ammoniumchlorid | | 0,7 % |

b) Herstellung der Reagenzlösung :

Die Herstellung der Reagenzlösung erfolgt gemäß Beispiel 1.

c) Homogenisierung der Probe : erfolgt gemäß Beispiel 1.

d) Durchführung der Messung : erfolgt gemäß Beispiel 1.

### Beispiel 4 (Vergleichsbeispiel)

Beispiel 4 entspricht Beispiel 3 mit dem einzigen Unterschied, daß die Homogenisierlösung kein Detergenz (Lauryl-trimethyl-ammoniumchlorid) enthält.

Die Ergebnisse einer vergleichenden Messung von identischen Proben mit einem Reagens gemäß Beispiel 3 und Beispiel 4 sind im folgenden gegenübergestellt.

| Probe Nr. | Ohne Detergenz (Beispiel 4) mE/min. | Mit Detergenz (Beispiel 3) mE/min. |
|---|---|---|
| 1 | 31 | 181 |
| 2 | 8 | 68 |
| 3 | 6 | 42 |
| 4 | 8 | 68 |
| 5 | 20 | 159 |

### Beispiel 5

a) Die Herstellung der Homogenisierlösung erfolgt gemäß Beispiel 1.

b) Herstellung der Reagenslösung : Es wird eine wäßrige Lösung mit folgenden Bestandteilen hergestellt :

| | |
|---|---|
| Ala-Ala-Phe-p-nitroanilid | 2 mmol/l |
| NaCl | 250 mmol/l |
| $CaCl_2$ | 20 mmol/l |
| Tris/HCl-Puffer, pH 9,0 | 60 mmol/l |

c) Homogenisierung der Probe : 200 mg Stuhlprobe werden mit 10 ml Homogenisierlösung versetzt und mit einem geeigneten Gerät zu einer feinen Suspension verarbeitet.

d) Durchführung der Messung : In eine 1 cm-Küvette werden 1 ml der Reagenzlösung pipettiert, auf 25 °C temperiert und mit 200 µl Probenhomogenisat versetzt. Nach kurzer Durchmischung wird die Extinktionszunahme bei 405 nm bestimmt.

### Beispiel 6 (Vergleichsbeispiel)

Beispiel 6 entspricht Beispiel 5 mit dem einzigen Unterschied, daß die Homogenisierlösung kein Detergenz enthält.

Das Ergebnis einer identischen Probe gemäß Beispiel 5 und Beispiel 6 ist im folgenden gegenübergestellt :

| Ohne Detergens (Beispiel 6) mE/min | Mit Detergens (Beispiel 5) mE/min |
|---|---|
| 31 | 45 |

Beispiel 7

a) Herstellung der Homogenisierlösung erfolgt gemäß Beispiel 1.

b) Herstellung der Reagenslösung : Es wird eine wäßrige Lösung mit folgenden Bestandteilen hergestellt :

| | |
|---|---|
| 3-Carbomethoxypropionyl-L-arginyl-L-prolyl-L-tyrosin-p-nitroanilid-hydrochlorid | 0,5 mMol/l |
| NaCl | 250 mMol/l |
| $CaCl_2$ | 20 mMol/l |
| Tris/HCl-Puffer, pH 9,0 | 60 mMol/l |

c) Homogenisierung und Messung erfolgt gemäß Beispiel 1.

Beispiel 8 (Vergleichsbeispiel)

Beispiel 8 entspricht Beispiel 7 mit dem einzigen Unterschied, daß die Homogenisierlösung kein Detergens enthält. Das Ergebnis der Messung einer identische Probe gemäß Beispiel 7 und Beispiel 8 ist im folgenden gegenübergestellt :

| Ohne Detergens (Beispiel 8) mE/min | Mit Detergens (Beispiel 7) mE/min |
|---|---|
| 19 | 148 |

Beispiel 9

Bestimmung von Trypsin

a) Die Herstellung der Homogenisierlösung erfolgt gemäß Beispiel 1.

b) Herstellung der Reagenzlösung : Es wird eine wäßrige Lösung mit folgenden Bestandteilen hergestellt :

| | |
|---|---|
| Carbobenzoxy-L-valyl-L-glycyl-L-arginin-p-nitroanilid-acetat | 5 mmol/l |
| NaCl | 250 mmol/l |
| $CaCl_2$ | 20 mmol/l |
| Tris/HCl-Puffer, pH 9,0 | 60 mmol/l |

c) Homogenisierung und d) Messung erfolgt gemäß Beispiel 1.

Beispiel 10 (Vergleichsbeispiel)

Beispiel 10 entspricht Beispiel 9 mit dem einzigen Unterschied, daß die Homogenisierlösung kein Detergenz enthält. Das Ergebnis der Messung mit einer identischen Probe gemäß Beispiel 9 und Beispiel 10 ist im folgenden gegenübergestellt :

| Ohne Detergenz (Beispiel 10) mE/min | Mit Detergenz (Beispiel 9) mE/min |
|---|---|
| ca. 3 | 8 |

Beispiel 11

Ein Aliquot der Stuhlprobe wird durch eine geeignete Vorrichtung derart dosiert, daß sich die Probe in einem Raum befindet, der auf einer Seite von einem Filterpapier oder ähnlichem abgegrenzt wird. Hierzu sind z. B. verschiedene kommerzielle Vorrichtungen zur Bestimmung von Blut im Stuhl wie Hemo FEC® oder Feca-Nostic® nach Austausch des entsprechenden Filterpapiers geeignet.

Das Filterpapier wird mit 50 μl einer wäßrigen Solubilisierungslösung bestehend aus :

| | |
|---|---|
| Lauryltrimethylamoniumchlorid | 50 g/l (5 %) |
| NaCl | 500 mmol/l |
| CaCl$_2$ | 100 mmol/l |

beträufelt, wobei das Chymotrypsin einer Grenzfläche solubilisiert wird und von dem Filterpapier aufgenommen wird. Anschließend wird das Filterpapier mit 50 μl einer Reagenzlösung folgender Zusammensetzung beträufelt :

| | |
|---|---|
| Succ-Ala-Ala-Pro-Phe-pNA | 2 mmol/l |
| N-α-Naphthyläthylendiamin | 5 g/l |
| Natriumnitrit | 1 g/l |
| Puffer Tris/HCl pH 9,0 | 100 mmol/l |

Nach 5 Minuten Reaktionszeit wird das Papier mit einem Tropfen Trichloressigsäure (3,2 mmol/l) versetzt, wobei sich bei Anwesenheit von Chymotrypsin in der Probe eine intensiv violette Färbung, deren Ausmaß von der Enzymmenge abhängt, ausbildet.

## Beispiel 12 (Vergleichsbeispiel)

Beispiel 11 wird wiederholt mit dem einzigen Unterschied, daß die zur Solubilisierung des Enzyms verwandte Lösung kein Detergenz enthält. Es entwickelt sich keine sichtbare Färbung.

## Ansprüche

1. Verfahren zur Bestimmung der Aktivität von Chymotrypsin oder Trypsin im Stuhl durch Messung der Geschwindigkeit der Spaltung eines geeigneten Substrats durch eine Stuhlsuspension in wäßrigem oder wäßrig-organischem Medium, dadurch gekennzeichnet, daß man den Stuhl in Gegenwart eines oberflächenaktiven Mittels suspendiert.

2. Verfahren nach Anspruch 1, dadurch gekennzeichnet, daß man die bei einer Substratspaltung freigesetzte Aminosäure bestimmt.

3. Verfahren nach Anspruch 2, dadurch gekennzeichnet, daß man die freigesetzte Aminosäure durch Titration mit einer Base bestimmt.

4. Verfahren nach Anspruch 1, dadurch gekennzeichnet, daß man die Geschwindigkeit der Spaltung des Substrats photometrisch bestimmt.

5. Verfahren nach einem der Ansprüche 1 bis 4, dadurch gekennzeichnet, daß man die Messung mit der Stuhlsuspension oder mit dem durch Zentrifugation der Suspension erhaltenen Zentrifugationsüberstand durchführt.

6. Verfahren nach einem der Ansprüche 1 bis 5, dadurch gekennzeichnet, daß man die Bestimmung manuell oder mit einem automatischen Analysengerät durchführt.

7. Verfahren nach einem der Ansprüche 1 bis 6, dadurch gekennzeichnet, daß man als oberflächenaktives Mittel ein kationisches oder nichtionisches Tensid verwendet.

8. Verfahren nach Anspruch 7, dadurch gekennzeichnet, daß man als oberflächenaktives Mittel ein quartäres Ammoniumsalz oder ein Alkylpyridiniumsalz verwendet, und insbesondere Lauryl-trimethyl-ammoniumchlorid.

9. Verfahren nach einem der Ansprüche 1 bis 8, dadurch gekennzeichnet, daß die Konzentration des oberflächenaktiven Mittels in der zur Suspension des Stuhls verwendeten Homogenisierlösung etwa 0,02 bis etwa 10 Gew.-%, vorzugsweise 0,5 bis 5 Gew.-%, beträgt.

10. Verfahren nach einem der Ansprüche 1 bis 9, dadurch gekennzeichnet, daß die zur Suspension des Stuhls verwendete Homogenisierlösung neben dem oberflächenaktiven Mittel ein oder mehrere wasserlösliche Salze enthält.

11. Verfahren nach Anspruch 10, dadurch gekennzeichnet, daß die Homogenisierlösung Natriumsalz oder/und Calciumsalz enthält.

12. Verfahren nach einem der Ansprüche 10 oder 11, dadurch gekennzeichnet, daß die Ionenstärke 20 bis 1 000 mVal/l beträgt.

13. Verfahren nach einem der Ansprüche 1 bis 12, dadurch gekennzeichnet, daß man Chymotrypsin bestimmt.

14. Verfahren nach Anspruch 13, dadurch gekennzeichnet, daß man als Substrat Ala-Ala-Phe-pNA, Succ-Ala-Ala-Pro-Phe-pNA oder MeO-Succ-Arg-Pro-Tyr-pNA verwendet.

7

15. Verfahren nach einem der Ansprüche 1 bis 12, dadurch gekennzeichnet, daß man Trypsin bestimmt.

16. Verfahren nach Anspruch 15, dadurch gekennzeichnet, daß man als Substrat Carbobenzoxy-Val-Gly-Arg-pNA-acetat verwendet.

17. Reagens zur Durchführung des Verfahrens nach Anspruch 1 bis 16, gekennzeichnet durch einen Gehalt an oberflächenaktivem Mittel, Enzymsubstrat, wasserlöslichem Salzwasser und einem pH-Wert von 7 bis 11.

18. Reagens nach Anspruch 17, dadurch gekennzeichnet, daß es

0,02 bis 10 Gew.-% oberflächenaktives Mittel,
0,05 bis 5 mMol/l Enzymsubstrat,
20 bis 1 000 mVal/l wasserlösliches Salz und
10 bis 1 000 mMol/l Puffer, pH 7 bis 11,

enthält.

19. Reagens nach Anspruch 18, dadurch gekennzeichnet, daß es

0,5 bis 5 Gew.-% oberflächenaktives Mittel,
0,2 bis 2 mMol/l Substrat,
100 bis 1 000 mMol/l NaCl oder/und
20 bis 500 mMol/l $CaCl_2$ und
50 bis 200 mMol/l Tris-Puffer, pH 8 bis 10,

enthält.

## Claims

1. Process for the determination of the activity of chymotrypsin or trypsin in faeces by measurement of the velocity of the splitting of a suitable substrate by a faecal suspension in aqueous or aqueous-organic medium, characterised in that one suspends the faeces in the presence of a surface-active agent.

2. Process according to claim 1, characterised in that one determines the amino acid liberated in the case of a substrate splitting.

3. Process according to claim 2, characterised in that one determines the liberated amino acid by titration with a base.

4. Process according to claim 1, characterised in that one determines the velocity of the splitting of the substrate photometrically.

5. Process according to one of claims 1 to 4, characterised in that one carries out the measurement with the faecal suspension or with the centrifugal supernatant obtained by centrifuging of the suspension.

6. Process according to one of claims 1 to 5, characterised in that one carries out the determination manually or with an automatic analysis apparatus.

7. Process according to one of claims 1 to 6, characterised in that as surface-active agent one uses a cationic or non-ionic tenside.

8. Process according to claim 7, characterised in that as surface-active agent, one uses a quaternary ammonium salt or an alkylpyridinium salt and especially lauryl trimethyl ammonium chloride.

9. Process according to one of claims 1 to 8, characterised in that the concentration of the surface-active agent in the homogenising solution used for the suspension of the faeces amounts to about 0.02 to about 10 wt.%, preferably 0.5 to 5 wt.%.

10. Process according to one of claims 1 to 9, characterised in that the homogenising solution used for the suspension of the faeces contains, besides the surface-active agent, one or more water-soluble salts.

11. Process according to claim 10, characterised in that the homogenising solution contains sodium salt and/or calcium salt.

12. Process according to claim 10 or 11, characterised in that the ionic strength amounts to 20 to 1 000 mVal/l.

13. Process according to one of claims 1 to 12, characterised in that one determines chymotrypsin.

14. Process according to claim 13, characterised in that as substrate one uses Ala-Ala-Phe-pNA, Succ-Ala-Ala-Pro-Phe-pNA or MeO-Succ-Arg-Pro-Tyr-pNA.

15. Process according to one of claims 1 to 12, characterised in that one determines trypsin.

16. Process according to claim 15, characterised in that as substrate one uses carbobenzoxy-Val-Gly-Arg-pNA acetate.

17. Reagent for the carrying out of the process according to claims 1 to 16, characterised by a content of surface-active agent, enzyme substrate, water-soluble salt, water and a pH value of 7 to 11.

18. Reagent according to claim 17, characterised in that it contains

0.02 to 10 wt.% of surface-active agent,
0.05 to 5 mMol/l. of enzyme substrate,
20 to 1 000 mVal/l. of water-soluble salt and
10 to 1 000 mVal/l. of buffer, pH 7 to 11.

19. Reagent according to claim 18, characterised in that it contains

```
0.5 to       5 wt.% of surface-active agent,
0.2 to       2 mMol/l. of substrate,
100   to 1 000 mMol/l. NaCl and/or
20   to   500 mMol/l. CaCl₂ and
50    to   200 mMol/l. tris buffer, pH 8 to 10.
```

**Revendications**

1. Procédé de détermination de l'activité de la chymotrypsine ou de la trypsine dans les fèces par mesure de la vitesse de coupure d'un substrat approprié par une suspension de fèces en milieu aqueux ou aqueux-organique, caractérisé en ce qu'on met en suspension les fèces en présence d'un agent tensio-actif.

2. Procédé suivant la revendication 1, charactérisé en ce qu'on dose l'aminoacide libéré lors d'une coupure du substrat.

3. Procédé suivant la revendication 2, caractérisé en ce qu'on dose l'aminoacide libéré par titrage avec une base.

4. Procédé suivant la revendication 1, caractérisé en ce qu'on détermine photométriquement la vitesse de coupure du substrat.

5. Procédé suivant l'une des revendications 1 à 4, caractérisé en ce qu'on effectue la mesure avec la suspension de fèces ou avec le liquide surnageant de la centrifugation obtenu par centrifugation de la suspension.

6. Procédé suivant l'une des revendications 1 à 5, caractérisé en ce qu'on effectue la détermination manuellement ou avec un appareil d'analyse automatique.

7. Procédé suivant l'une des revendications 1 à 6, caractérisé en ce qu'on utilise comme agent tensio-actif un agent tensio-actif cationique ou non ionique.

8. Procédé suivant la revendication 7, caractérisé en ce qu'on utilise comme agent tensio-actif un sel d'ammonium quaternaire ou un sel d'alcoylpyridinium, et en particulier du chlorure de lauryl-triméthyl-ammonium.

9. Procédé suivant l'une des revendications 1 à 8, caractérisé en ce que la concentration de l'agent tensio-actif dans la solution d'homogénéisation utilisée pour la mise en suspension des fèces est d'environ 0,02 à environ 10 % en poids, de préférence de 0,5 à 5 % en poids.

10. Procédé suivant l'une des revendications 1 à 9, caractérisé en ce que la solution d'homogénéisation utilisée pour la mise en suspension des fèces contient, en plus de l'agent tensio-actif, un ou plusieurs sels solubles dans l'eau.

11. Procédé suivant la revendication 10, caractérisé en ce que la solution d'homogénéisation contient un sel de sodium ou/et un sel de calcium.

12. Procédé suivant l'une des revendications 10 ou 11, caractérisé en ce que la force ionique est de 20 à 1 000 mval/l.

13. Procédé suivant l'une des revendications 1 à 12, caractérisé en ce qu'on dose la chymotrypsine.

14. Procédé suivant la revendication 13, caractérisé en ce qu'on utilise comme substrat de l'Ala-Ala-Phe-pNA, du Succ-Ala-Ala-Pro-Phe-pNA ou du MeO-Succ-Arg-Pro-Tyr-pNA.

15. Procédé suivant l'une des revendications 1 à 12, caractérisé en ce qu'on dose la trypsine.

16. Procédé suivant la revendication 15, caractérisé en ce qu'on utilise comme substrat du carbobenzoxy-Val-Gly-Arg-pNA-acétate.

17. Réactif pour effectuer le procédé suivant la revendication 1 à 16, caractérisé en ce qu'il contient un agent tensio-actif, un substrat d'enzyme, un sel soluble dans l'eau, de l'eau, et en ce que la valeur de son pH est de 7 à 11.

18. Réactif suivant la revendication 17, caractérisé en ce qu'il contient

```
0,02 à     10 % en poids d'agent tensio-actif,
0,05 à      5 mmoles/l de substrat d'enzyme,
20    à 1 000 mval/l de sel soluble dans l'eau et
10    à 1 000 mmoles/l de tampon, pH 7 à 11.
```

19. Réactif suivant la revendication 18, caractérisé en ce qu'il contient :

```
0,5 à      5 % en poids d'agent tensio-actif,
0,2 à      2 mmoles/l de substrat,
100    à 1 000 mmoles/l de Nacl ou/et
20    à   500 mmoles/l de Cacl₂ et
50    à   200 mmoles/l de tampon tris, pH 8 à 10.
```